# EUROPÄISCHE PATENTSCHRIFT

(11) **EP 1 807 225 B1**
(45) Veröffentlichungstag und Bekanntmachung des Hinweises auf die Patenterteilung: **24.02.2016**
(21) Anmeldenummer: 05817092.9
(22) Anmeldetag: 03.11.2005
(51) Int. Cl.: B09B 3/00, C05F 17/00

(54) **ABBAUVERFAHREN VON BIOGENEM MATERIAL, DAZUGEHÖRIGE BIOGASANLAGE SOWIE DEREN VERWENDUNG**
METHOD FOR DECOMPOSING BIOGENIC MATERIAL, CORRESPONDING BIOGAS PLANT AND ITS USE
PROCÉDÉ DE DÉCOMPOSITION DE MATÉRIAU BIOGÈNE, INSTALLATION À BIOGAZ CORRESPONDANTE ET SON UTILISATION

(30) Priorität: 03.11.2004 DE 102004053615
(43) Veröffentlichungstag der Anmeldung: 18.07.2007
(73) Patentinhaber: Brandenburgische Technische Universität Cottbus-Senftenberg, 03046 Cottbus (DE)
(72) Erfinder: BUSCH, Günter, 01462 Cossebaude (DE); SIEBER, Marko, 03149 Forst (DE)
(74) Vertreter: Schubert, Klemens
(86) Internationale Anmeldenummer: PCT/DE2005/001990
(87) Internationale Veröffentlichungsnummer: WO 2006/048008

(56) Entgegenhaltungen:
- EP-A- 0 659 696
- DE-A1- 3 545 679
- DE-A1- 19 623 163
- DE-A1- 19 846 336
- DE-A1- 19 909 328
- US-A- 4 400 195
- US-A- 5 269 634
- US-A- 6 110 727

## Beschreibung

Die Erfindung betrifft ein Abbauverfahren von biogenem Material und eine dazugehörige Biogasanlage sowie deren Verwendung.

Die Vergärung von biologischen Stoffen ist ein seit langem bekannter Prozess. Aufgrund von verschiedensten Entwicklungen wurden ein-, zwei- oder mehrstufige Verfahren entwickelt. Neben der aus der Güllevergärung entwickelten Nassvergärung wird ebenfalls die Trockenvergärung praktiziert.

Das Prinzip der zweistufigen Trocken-Nass-Vergärung wurde erstmals von Gosh 1978 beschrieben. Dabei wurden Abfälle in einen anaeroben Reaktor perkoliert. Das Perkolationswasser wird anschließend in einem Methanreaktor zu Biogas vergoren. Das Verfahren wurde von Rijkens und Hofank (US 4,400,195) in den 80er Jahren für organische Abfälle weiterentwickelt und patentiert.

Dieses Verfahren wurde zweimal in die Praxis umgesetzt, im ANM-Verfahren in Ganderkesee und im Prethane-Rudad-Verfahren in Breda.

Wellinger und Suter führten ebenfalls in den 80er Jahren Versuche mit Festmist durch und Widmer mit Markt- und Schlachtabfällen. Dabei wurde der Perkolator auch im aeroben Milieu betrieben.

Die neueste Anlage, welche nach diesem Verfahren (für Restabfälle) entwickelt wurde, sind das ISKA^{®}-Perkolationsverfahren in Sansenheck und das BIOPERCOLAT^{®}-Verfahren (DE 198 46 336 A1). Dabei wird der Abfall nach einer mechanischen Vorbehandlung (zum Beispiel Siebung, Metallabscheidung) in einem Perkolator hydrolysiert. Der Perkolator ist dabei mit einem Rührwerk ausgestattet, so dass die Abfälle kontinuierlich durch den Reaktor transportiert werden. Nach einer Verweilzeit von 2 bis 3 Tagen wird das Perkolat entwässert und für die weitere Behandlung beziehungsweise Ablagerung bereitgestellt. Das Perkolationswasser wird nach einer Sand- und Faserabscheidung in einem Methanreaktor anaerob zu Biogas vergoren. Das so gereinigte Wasser wird direkt bzw. nach einer Reinigung (zur Entstickung), als Perkolationswasser genutzt.

Weiterhin ist aus der nächstliegenden Stand der Technik, US 6 110 727 A, ein Verfahren und eine Vorrichtung zur biologischen Behandlung organischer Materialien bekannt. Offenbart wird die Verwendung eines horizontalen Reaktors, in welchem das organische Material mittels geeigneter Vorrichtungen durchmischt wird. Das Material wird in einer aeroben Reaktionsstufe ausgelaugt und die Auslaugflüssigkeit wird direkt im Anschluss an den Durchtritt durch das Material einer anaeroben Reaktionsstufe zur Biogasgewinnung unterworfen. Nach der Gewinnung des Biogases aus der Auslaugflüssigkeit, einem Reinigungsschritt und Belüften der Flüssigkeit kann diese wieder zum weiteren Auslaugen auf das Material aufgebracht werden.

In der DE 199 09 328 A1 wird ein Abfallverwertungsverfahren offenbart, bei welchem ein Ersatzbrennstoff gewonnen wird. Ein unbedingt erforderlicher Schritt dieses Verfahrens ist die Zerkleinerung der Abfallmasse, weil die reine Perkolation des Abfallmaterials den Zwecken dieses Verfahrens nicht genügt. Bei diesem Abfallverwertungsverfahren entstehende Auswaschflüssigkeit kann einer Abwasserreinigungsanlage mit Bioreaktor zugeführt werden. Dort kann daraus Biogas gewonnen werden und die Flüssigkeit im Anschluss an diese anaerobe Reaktionsstufe wieder auf den Abfall aufgebracht werden.

Die DE 35 45 679 A1 offenbart ein Verfahren zur Entsorgung organischen Hausmülls. Auch dieses Verfahren umfasst eine aerobe Stufe der Auslaugung und eine anaerobe Stufe der Biogasgewinnung. Wie bei den Schutzrechten US 6 110 727 A und DE 199 09 328 A1 kann auch bei diesem Verfahren die Auslaugflüssigkeit wieder auf das organische Material rückgeführt werden. Allerdings wird auch hier vor der Rückführung auf das Material die Flüssigkeit einer anaeroben Reaktionsstufe unterworfen.

Der Erfindung liegt die Aufgabe zugrunde beim Abbau von biogenem Material eine bedarfsgerechte Steuerung der Biogaserzeugung zu ermöglichen.

Die Aufgabe wird durch ein Abbauverfahren gelost, dessen Merkmale in Hauptanspruch wiedergegeben sind. Vorteilhafte Ausgestaltungen sind in den abhängigen Ansprüchen gekennzeichnet.

Die Aufgabe der vorliegenden Erfindung wird durch ein Abbauverfahren gemäß Anspruch 1 gelöst, wobei man einen Perkolator mit biogenem Material beschickt,
eine Perkolationsflüssigkeit durch ein Sieb abtrennt und wieder auf das biogene Material sprüht,
überschüssige Perkolationsflüssigkeit in einen Pufferbehälter verbracht, von dort in einen Biogasreaktor gepumpt und zu Biogas vergärt, wobei man die im Biogasreaktor (6) gereinigte Perkolationsflüssigkeit als Abwasser in einen Speicherpufferbehälter überführt und wobei das Abwasser von dort wieder in den Perkolator überführbar ist.

Erfindungsgemäß ist somit ein Abbauverfahren von biogenem Material unter bedarfsgerechter Steuerung des Biogasanfalls, wobei man einen aerob betriebenen Perkolator mit biogenem Material beschickt,
Perkolationsflüssigkeit durch ein Sieb abtrennt und wieder auf das biogene Material sprüht,
überschüssige Perkolationsflüssigkeit in einen separaten Pufferbehälter verbringt, von dort in einen Biogasreaktor pumpt und zu Biogas vergärt, wobei man die im Biogasreaktor (6) gereinigte Perkolationsflüssigkeit als Abwasser in einen separaten Speicherpufferbehälter überführt und von dort wieder in den Perkolator überführen kann.

Dieses Abbauverfahren hat den Vorteil, dass es auf einer einfachen Technik zum Abbau von biogenem Material basiert. Hierbei wird eine bedarfsgerechte Steuerung des Biogasanfalls ermöglicht und dadurch kann der Biogasbedarf, zum Beispiel für die Verstromung oder Wärmeerzeugung, in Spitzenzeiten bzw. Schwachlastzeiten entsprechend geregelt werden. Während bei bekannten Anlagen die Steuerung der Biogasproduktion nicht oder nur in engeren Perioden zur Verbrauchsanpassung erfolgt, kann mit dem erfindungsgemäßen Abbauverfahren eine rasche Anpassung an den aktuellen Bedarf erfolgen.

Als biogenes Material werden alle von Lebewesen stammenden Substanzen angesehen, insbesondere können dies Bioabfälle, Grünschnitt, Gewerbeabfälle, Lebensmittelabfälle, Siedlungsabfälle, landwirtschaftliche Abfälle, Küchenabfälle, nachwachsende Rohstoffe und ähnliche Stoffe sein.

Bevorzugt speichert man die Perkolationsflüssigkeit. Die bedarfsgerechte Steuerung des Biogasanfalls wird über entsprechend dimensionierte Pufferbehälter (max. 24 h Puffer) für die Perkolationsflüssigkeit möglich, da wegen der gefundenen verfahrenstechnischen Betriebsweise der Methanreaktionen die Ansprechzeit für die Biogasproduktion im Stundenbereich liegt. Die Funktion des Gasspeichers kann daher durch die Speicherung der Perkolationsflüssigkeit ersetzt werden.

In einer bevorzugten Ausführungsform der Erfindung beaufschlagt man diskontinuierlich einen Druckluftstoß durch perforierte Wände eines Siebbodens. Auf diese Weise lassen sich Verstopfungen des Siebbodens lösen bzw. lockern und es kann wieder ein freier Ablauf der Perkolationsflüssigkeit durch den Siebboden gewährleistet werden.

Im Perkolator herrscht bevorzugt Normaldruck. Ein Luftzutritt ist unschädlich. Ein Lufteintritt kann in den Perkolator kontinuierlich oder diskontinuierlich unabhängig vom Druckluftstoß erfolgen. Ferner ist es möglich, den Perkolator zu beheizen. Die Temperatur im Perkolator sollte dann ca. 30 °C betragen. Vorteilhafterweise wird der Perkolator aerob betrieben.

Die gesamte Flüssigkeit wird in dem System durch einen Kreislauf gepumpt. Die als Abwasser den Bioreaktor verlassende Pekolationsflüssigkeit führt man vorzugsweise diskontinuierlich ab und füllt mit frischer Flüssigkeit auf. Dadurch wird eine Anreicherung von Fremdstoffen im Kreislauf vermieden.

In dem Pufferbehälter und/oder in dem Speicherpufferbehälter scheidet man bevorzugt Sink- und/oder Schwimmstoffe ab. Dieser Vorgang kann durch die Verwendung der verschiedensten Abscheider bzw. Hochleistungsabscheider, wie zum Beispiel Zentrifugen, durchgeführt werden.

Das Vergären zu Biogas führt man bevorzugt mittels Bakterien durch. Dabei führt man die Vergärung unter Beteiligung einer Bakterienmatrix aus mehreren Bakterienstämmen durch. Der Biogasreaktor ist beheizbar, insbesondere außenbeheizbar. Damit kann im Biogasreaktor immer eine konstante Temperatur gehalten werden. Diese liegt entweder bei ca. 37 °C oder bei 55 °C.

Ferner wird die Erfindung durch eine Biogasanlage gemäß Anspruch 9 gelöst, wobei der Perkolator hier nach dem Garagenverfahren im aeroben Bereich aufgebaut ist.

Bevorzugt besteht die Biogasanlage aus mindestens zwei parallel geschalteten Perkolatoren. Dabei wird die Perkolationsflüssigkeit durch eine separate Pumpe pro Perkolator je nach Einsatzstoff kontinuierlich oder diskontinuierlich über das biogene Material versprüht. Es entsteht somit ein für jeden Perkolator separat betreibarer Flüssigkeitskreislauf. Eine Verbindung zwischen den einzelnen Perkolatoren ist möglich, um die biogenen Stoffe durch bestimmte Bakterienarten anzuimpfen.

Die Verwendung von mindestens zwei Perkolatoren hat weiterhin den Vorteil, dass eine Be- und Entladung von Feststoffen jederzeit möglich ist. Außerdem ist durch den modularen Aufbau eine Anpassung an den jeweiligen Stoffanfall bzw. die Energieabnahme möglich bzw. die substratspezifische Verweilzeit im Perkolator ist einzeln steuerbar. Wegen der aeroben Betriebsweise der Perkolatoren ist die Bildung von Methan und daher einer explosionsgefährdeten Atmosphäre im Perkolator nicht möglich.

Beim Abbau des biogenen Materials entstehen Säuren, daher sind die Perkolatoren bevorzugt säurefest. Die Perkolationsflüssigkeit löst jedoch die entstehenden Säuren und andere Stoffe auf und die Perkolationsflüssigkeit reichert sich mit leicht vergärbaren Stoffen an.

Es ist ebenfalls denkbar, dass ein extrem saurer Flüssigkeitsstrom zusammen mit der Perkolatorflüssigkeit im Biogasreaktor behandelt wird. Beispielsweise fällt in der Konservenindustrie einerseits ein hoher Abwasserstrom mit organischen Belastungen an, der meist stark sauer ist, andererseits gibt es aber auch feste Abfälle. Mit dem bedarfsgerechten Einsatz von festen Abfällen könnte der jahreszeitlich schwankende Anfall von saurem Abwasser ausgeglichen werden, so dass in Zeiten geringen Abwasseranfalls immer noch eine gute Biogasproduktion vorhanden ist.

Die Perkolatoren weisen einen Siebboden auf. Dieser dient dazu, um eine fest-flüssig Trennung vorzunehmen. Die abgetrennte Perkolationsflüssigkeit sammelt sich am Boden bzw. unter dem Siebboden und wird durch Pumpen kontinuierlich oder diskontinuierlich im Kreislauf über dem biogenen Material versprüht. Überschüssige Perkolationsflüssigkeit wird bei entsprechendem Füllstand in den Pufferbehälter gepumpt und von dort in den Biogasreaktor.

Die Temperatur im Perkolator sollte ungefähr 30 °C betragen, deshalb sind die Perkolatoren vorzugsweise beheizbar.

Es ist erfindungsgemäß bevorzugt, dass das Vergären zu Biogas mittels zugesetzter und/oder immobilisierter Bakterien erfolgt.

Der Biogasreaktor ist im Wesentlichen gasdicht und funktioniert nach einem der aus der Abwassertechnik üblichen Reaktorprinzipen (UASB- [Upflow anaerobic sludge bed], Schlammbett, -Festbettreaktor).

Ein Festbettbiogasreaktor kann als Pfropfenstromreaktor (Filter) betrieben werden, so dass sowohl die Verweilzeit im Perkolator als auch die Verweilzeit im Biogasreaktor definiert werden kann.

Biogas besteht aus Methan (CH₄) [50-85 Vol-%], Kohlendioxid (CO₂) [15-50 Vol-%] sowie Sauerstoff, Stickstoff und Spurengasen (u.a. Schwefelwasserstoff). Mit dem erfindungsgemäße Abbauverfahren erzeugt man ein Biogas mit einem hohen Methananteil von zwischen 65 und 80 Vol-%. Es kann u.a. direkt für Heizzwecke oder mittels eines Blockheizkraftwerks zur gekoppelten Produktion von Strom und Wärme genutzt werden. Die Erzeugung des Gases erfolgt durch anaerobe Vergärung organischer Stoffe.

Zur Erhöhung des Biogasertrags kommen häufig Co-Fermentate zum Einsatz (zum Beispiel nachwachsende Rohstoffe oder Abfälle aus der Lebensmittelindustrie). Das vergorene organische Material kann anschließend als hochwertiger Dünger landbaulich verwertet werden.

Nach dem Vergärungsprozess verlässt das gereinigte Perkolationswasser als Abwasser den Methanreaktor und wird im Speicherpufferbehälter zwischengespeichert und kann von dort in die Perkolatoren zurückgeführt werden.

Damit im Biogasreaktor immer eine konstante Temperatur gehalten werden kann, ist der Biogasreaktor vorzugsweise beheizbar. Die Temperatur liegt dabei entweder bei ungefähr 37 °C oder bei ungefähr 55 °C.

In einer bevorzugten Ausführungsform der Erfindung ist der Speicherpufferbehälter belüftbar. Dies hat den Vorteil, dass Bakterien aus dem Biogasreaktor abgetötet werden, so dass eine Animpfung der Hydrolyse mit Methanbakterien und dadurch eine Produktion von Biogas in den Perkolatoren ausgeschlossen wird.

Damit eine Akkumulierung verschiedenster Stoffe im Abwasser vermieden werden kann, wird ein Teil des Kreislaufwassers abgeführt und mit Frischwasser aufgefüllt.

Weiterhin wird die Aufgabe der Erfindung durch die Verwendung der Biogasanlage zur Herstellung von Biogas und der Speicherung von Perkolationsflüssigkeit gemäß Anspruch 16 gelöst.

Durch Mischung mit Luft kann Biogas leicht zu explosiven Gemischen führen, daher unterliegt die Herstellung und Speicherung besonderen Sicherheitsvorschriften.

Eine solche Explosionsgefahr kann durch das erfindungsgemäße Verfahren stark vermindert werden, da die Perkolatoren auf einer aeroben Betriebsweise beruhen und somit die Bildung von Methan unterbunden wird. Des Weiteren ist eine Speicherung von Biogas nicht notwendig, da man lediglich die Perkolationsflüssigkeit speichert.

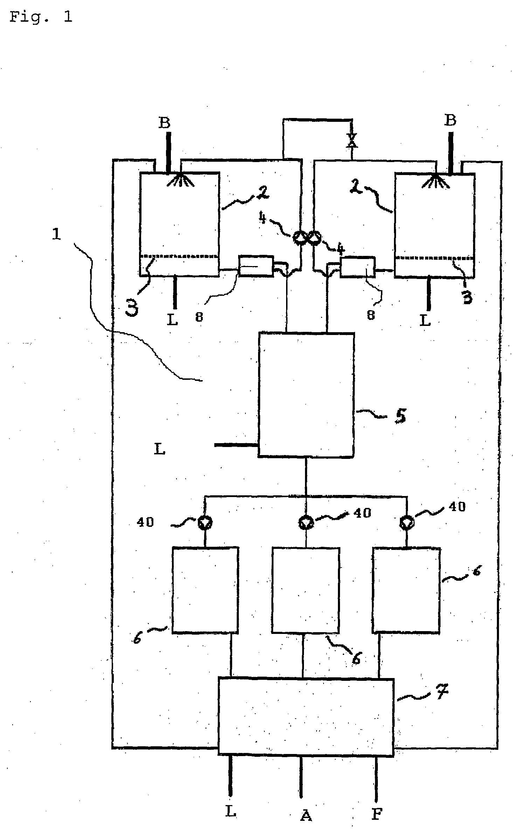

Im folgenden wird die Erfindung anhand einer Figur näher beschrieben. Im einzelnen zeigt
- Figur 1: eine schematische Darstellung einer erfindungsgemäßen Biogasanlage.

Die Figur 1 zeigt eine schematische Darstellung einer Biogasanlage 1 zur bedarfsgerechten Herstellung von Biogas.

Die Perkolatoren 2 sind im Garagenverfahren aufgebaut, d.h. sie bestehen als eine Art Container bzw. sonstigen Raum mit säurefester Auskleidung. Sie sind derart gestaltet, dass sie mit üblicher Technik (zum Beispiel mit Radladern) beschickt und entleert werden können. Der Boden und/oder die Wände sind außerdem mit Siebböden 3 ausgestattet, so dass an diesen Stellen eine fest-flüssig Trennung stattfinden kann. Zur Vermeidung von Verstopfungen oder um das Material aufzulockern kann diskontinuierlich ein Druckluftstoß durch diese perforierten Wände aufgegeben werden. Ansonsten arbeiten die Perkolatoren 2 bei Umgebungsdruck und ein Luftzutritt ist unschädlich und kann auch unabhängig vom Druckluftstoß kontinuierlich oder diskontinuierlich erfolgen. Die Perkolationsflüssigkeit sammelt sich am Boden bzw. unter den Siebböden 3 und wird über je eine Pumpe 4 pro Perkolator 2 kontinuierlich oder diskontinuierlich im Kreislauf über dem biogenen Material versprüht. Die Perkolationsflüssigkeit löst die entstehenden Säuren und andere Stoffe auf dem Festbett. Somit reichert sich die Perkolationsflüssigkeit mit leicht vergärbaren Stoffen an.

Überschüssige Perkolationsflüssigkeit wird bei entsprechendem Füllstand in den Pufferbehälter 5 gepumpt. Fest- und/oder Schwebstoffe werden gegebenenfalls zuvor im Abscheider 8 abgetrennt. Von dort wird die Perkolationsflüssigkeit mittels der Pumpen 40 gasdicht in den Biogasreaktor 6 gepumpt. Dieser funktioniert nach einem der aus der Abwassertechnik üblichen Reaktionsprinzipen (UASB-, Schlammbett, -festbettreaktor). In den Biogasreaktoren 6 wird die Perkolationsflüssigkeit schnell zu Biogas vergoren. Die so gereinigte Perkolationsflüssigkeit verlässt als Abwasser die Biogasreaktoren 6 und wird im Speicherpufferbehälter 7 zwischengespeichert, bevor es je nach Bedarf in die Perkolatoren 2 zurückgeführt wird und dort den Flüssigkeitsspiegel ansteigen lässt. Damit wird die Flüssigkeit immer im Kreislauf geführt. Damit eine Akkumulation verschiedener Stoffe in der Flüssigkeit vermieden wird, wird ein Teil des Kreislaufflüssigkeit diskontinuierlich abgeführt und mit frischer Flüssigkeit (Wasser) aufgefüllt.

Der Pufferbehälter 5 und der Speicherpufferbehälter 7 dienen neben der Speicherwirkung ebenfalls als Abscheider für Sink- oder Schwimmstoffe, die diskontinuierlich entnommen werden. Zusätzlich besteht die Möglichkeit, durch Einsatz von Hochleistungsabscheidern (zum Beispiel Zentrifugen) weitere unerwünschte Stoffe aus den einzelnen Pufferbehältern 5 und/oder Speicherpufferbehältern 7 abzuscheiden.

Die Speicherpufferbehälter 7 können bei Bedarf ebenfalls belüftet werden, um den weiteren biologischen Abbau bzw. die Abscheidung von unerwünschten Stoffen, wie zum Beispiel Ammonium, im Speicherpufferbehälter 7 zu begünstigen. Durch die Belüftung der Speicherpufferbehälter 7 werden außerdem die aus dem Bioreaktor ausgetragenen Bakterien weitestgehend abgetötet, so dass eine Animpfung der Hydrolyse mit Methanbakterien und somit eine Produktion von Biogas in den Perkolatoren 2 ausgeschlossen ist.

Die Biogasanlage 1 kann erfindungsgemäß insbesondere derart gefahren werden, dass der aerobe Perkolationskreislauf und der anaerobe Biogasproduktionskreislauf strikt voneinander getrennt sind. Damit ist sichergestellt, dass im Bereich der Perkolatoren 2 und/oder in Bereich der Speicherpufferbehälter 7 keine sicherheitsrelevante Menge an freiem Biogas (Methan) vorliegt. Dies führt zu einer verbesserten Betriebssicherheit der gesamten Anlage.

### Bezugszeichenliste:

- 1: Biogasanlage
- 2: Perkolator
- 3: Siebboden
- 4: Pumpe
- 5: Pufferbehälter
- 6: Biogasreaktor
- 7: Speicherpufferbehälter
- 8: Abscheider
- 40: Pumpe
- A: Abwasser
- B: Biogenes Material
- F: Frischwasser
- L: Luft

## Patentansprüche

1. Abbauverfahren von biogenem Material,
wobei man einen Perkolator (2) mit biogenem Material beschickt,
Perkolationsflüssigkeit durch ein Sieb abtrennt und die abgetrennte Perkolationsflüssigkeit durch Pumpen kontinuierlich oder diskontinuierlich im Kreislauf wieder über dem biogenen Material versprüht,
überschüssige Perkolationsflüssigkeit in einen Pufferbehälter (5) verbringt, von dort in einen Biogasreaktor (6) pumpt und zu Biogas vergärt, wobei man
die im Biogasreaktor (6) gereinigte Perkolationsflüssigkeit als Abwasser in einen Speicherpufferbehälter (7) überführt und wobei das Abwasser von dort wieder in den Perkolator (2) überführbar ist.

2. Abbauverfahren nach Anspruch 1, **dadurch gekennzeichnet, dass** man die Perkolationsflüssigkeit speichert.

3. Abbauverfahren nach einem der Ansprüche 1 oder 2, **dadurch gekennzeichnet, dass** man diskontinuierlich einen Druckluftstoß durch perforierte Wände eines Siebbodens (3) beaufschlagt.

4. Abbauverfahren nach einem der Ansprüche 1 bis 3, **dadurch gekennzeichnet, dass** im Perkolator (2) Normaldruck herrscht.

5. Abbauverfahren nach einem der Ansprüche 1 bis 4, **dadurch gekennzeichnet, dass** man den Perkolator (2) aerob betreibt.

6. Abbauverfahren nach einem der Ansprüche 1 bis 5, **dadurch gekennzeichnet, dass** man einen Teil des Abwassers diskontinuierlich abführt und mit frischer Flüssigkeit auffüllt.

7. Abbauverfahren nach einem der Ansprüche 1 bis 6, **dadurch gekennzeichnet, dass** man in dem Pufferbehälter (5) und/oder in dem Speicherpufferbehälter (7) Sink- und/oder Schwimmstoffe abscheidet.

8. Abbauverfahren nach einem der Ansprüche 1 bis 7, **dadurch gekennzeichnet, dass** man das Vergären zu Biogas mittels zugesetzter und/oder immobilisierter Bakterien durchführt.

9. Biogasanlage (1) für ein Verfahren nach einem der vorhergehenden Ansprüche, bestehend aus mindestens einem Perkolator (2) mit Siebboden (3) und Pumpe (4),
einem zwischen Pumpe (4) und Siebboden angeordneten Abscheider (8), wobei die Pumpe (4) innerhalb einer Leitung angeordnet ist, welche den Bereich des Perkolators (2) unterhalb des Siebbodens (3) über dem Abscheider (8) mit dem oberen Bereich des Perkolators verbindet,
einem Pufferbehälter (5), mindestens einem Biogasreaktor (6) und mindestens einem Speicherpufferbehälter (7).

10. Biogasanlage (1) nach Anspruch 9, **dadurch gekennzeichnet, dass** sie aus mindestens zwei parallel geschalteten Perkolatoren (2) besteht.

11. Biogasanlage (1) nach einem der Ansprüche 9 oder 10,
**dadurch gekennzeichnet, dass** die Perkolatoren (2) säurefest sind.

12. Biogasanlage (1) nach einem der Ansprüche 9 bis 11, **dadurch gekennzeichnet, dass** die Perkolatoren (2) beheizbar sind.

13. Biogasanlage (1) nach einem der Ansprüche 9 bis 12, **dadurch gekennzeichnet, dass** der Biogasreaktor (6) gasdicht ist.

14. Biogasanlage (1) nach einem der Ansprüche 9 bis 13, **dadurch gekennzeichnet, dass** der Biogasreaktor (6) beheizbar ist.

15. Biogasanlage (1) nach einem der Ansprüche 9 bis 14, **dadurch gekennzeichnet, dass** der Speicherpufferbehälter (7) belüftbar ist.

16. Verwendung der Biogasanlage nach einem der Ansprüche 9 bis 15 zur Herstellung von Biogas und Speicherung von Perkolationsflüssigkeit.

## Claims

1. A method for decomposing biogenic material, wherein a percolator (2) is loaded with biogenic material,
percolation liquid is separated through a sieve and the separated percolation liquid is again sprayed by means of pumps continuously or discontinuously onto the biogenic material.
excess percolation liquid is introduced into a buffer tank (5), from there pumped into a biogas reactor (6) and fermented to biogas, wherein the in the biogas reactor cleaned percolation liquid is transferred as wastewater into a storage buffer tank (7) and wherein the wastewater can be transferred from there again into the percolator (2).

2. The decomposing method according to claim 1, further **characterized in that** the percolation liquid is stored.

3. The decomposing method according to one of claims 1 or 2, further **characterized in that** a shock of compressed air is impressed discontinuously through perforated walls of a sieve bottom (3).

4. The decomposing method according to one of claims 1 to 3, further **characterized in that** normal pressure prevails in percolator (2).

5. The decomposing method according to one of claims 1 to 4, further **characterized in that** percolator (2) is driven aerobically.

6. The decomposing method according to one of claims 1 to 5, further **characterized in that** a part of the wastewater is discharged discontinuously and fresh liquid is introduced.

7. The decomposing method according to one of claims 1 to 6, further **characterized in that** sinking and/or floating substances are separated from buffer tank (5) and/or from storage buffer tank (7).

8. The decomposing method according to one of claims 1 to 7, further **characterized in that** the fermenting to biogas is conducted by means of added and/or immobilized bacteria.

9. A biogas plant (1) for a method according to one of the preceding claims, consisting of at least one percolator (2) with a sieve bottom (3) and a pump (4), a separator (8) arranged between pump (4) and sieve bottom (3), wherein the pump (4) is arranged inside a pipe that connects the part of the percolator (2) underneath the sieve bottom (3) through the separator (8) with the upper part of the percolator,
a buffer tank (5), at least one biogas reactor (6), and at least one storage buffer tank (7).

10. The biogas plant (1) according to claim 9, further **characterized in that** it consists of at least two parallelly connected percolators (2).

11. The biogas plant (1) according to one of claims 9 or 10, further **characterized in that** the percolators (2) are acid-resistant.

12. The biogas plant (1) according to one of claims 9 to 11, further **characterized in that** the percolators (2) are heatable.

13. The biogas plant (1) according to one of claims 9 to 12, further **characterized in that** the biogas reactor (6) is gas-tight.

14. The biogas plant (1) according to one of claims 9 to 13, further **characterized in that** the biogas reactor (6) is heatable.

15. The biogas plant (1) according to one of claims 9 to 14, further **characterized in that** the storage buffer tank (7) can be aerated.

16. A use of the biogas plant according to one of claims 9 to 15 for the production of biogas and for the storage of percolation liquid.

## Revendications

1. Procédé de décomposition de matière biogène,
où un percolateur (2) est chargé de matière biogène,
un liquide de percolation est séparé par tamis et le liquide de percolation séparé est recyclé et ré-aspergé par des pompes sur la matière biogène, de manière continue ou discontinue,
le liquide de percolation en excédent est acheminé vers un récipient tampon (5), dont il est pompé vers un réacteur à biogaz (6) et méthanisé, où le
liquide de percolation épuré dans le réacteur à biogaz (6) est conduit en tant qu'eaux usées vers un réservoir tampon (7), et où les eaux usées peuvent être reconduites de ce dernier vers le percolateur (2).

2. Procédé de décomposition selon la revendication 1, **caractérisé en ce que** le liquide de percolation est stocké.

3. Procédé de décomposition selon la revendication 1 ou la revendication 2, **caractérisé en ce qu'**un jet d'air comprimé est pulsé de manière discontinue par les parois perforées d'un fond de tamis (3).

4. Procédé de décomposition selon l'une des revendications 1 à 3, **caractérisé en ce que** l'intérieur du percolateur (2) est soumis à la pression atmosphérique.

5. Procédé de décomposition selon l'une des revendications 1 à 4, **caractérisé en ce que** le percolateur (2) est mis en service de manière aérobie.

6. Procédé de décomposition selon l'une des revendications 1 à 5, **caractérisé en ce qu'**une partie des eaux usées est évacuée de manière discontinue, un liquide frais étant versé.

7. Procédé de décomposition selon l'une des revendications 1 à 6, **caractérisé en ce que** des matières en suspension et/ou flottantes sont séparées dans le récipient tampon (5) et/ou dans le réservoir tampon (7).

8. Procédé de décomposition selon l'une des revendications 1 à 7, **caractérisé en ce que** la méthanisation est effectuée au moyen de bactéries ajoutées et/ou immobilisées.

9. Installation de méthanisation (1) pour un procédé selon l'une des revendications précédentes, composée d'au moins un percolateur (2) avec un fond de tamis (3) et une pompe (4), un séparateur (8) disposé entre la pompe (4) et le fond de tamis, la pompe (4) étant disposée à l'intérieur d'une conduite, laquelle relie par le séparateur (8) la partie du percolateur (2) en dessous du fond de tamis (3) à la partie supérieure du percolateur,
un récipient tampon (5), au moins un réacteur à biogaz (6) et au moins un réservoir tampon (7).

10. Installation de méthanisation (1) selon la revendication 9, **caractérisée en ce que** celle-ci est composée d'au moins deux percolateurs (2) montés en parallèle.

11. Installation de méthanisation (1) selon l'une des revendications 9 ou 10, **caractérisée en ce que** les percolateurs (2) sont résistants aux acides.

12. Installation de méthanisation (1) selon l'une des revendications 9 à 11, **caractérisée en ce que** les percolateurs (2) peuvent être chauffés.

13. Installation de méthanisation (1) selon l'une des revendications 9 à 12, **caractérisée en ce que** le réacteur à biogaz (6) est étanche aux gaz.

14. Installation de méthanisation (1) selon l'une des revendications 9 à 13, **caractérisée en ce que** le réacteur à biogaz (6) peut être chauffé.

15. Installation de méthanisation (1) selon l'une des revendications 9 à 14, **caractérisée en ce que** le réservoir tampon (7) peut être aéré.

16. Utilisation de l'installation de méthanisation selon l'une des revendications 9 à 15 pour la production de biogaz et le stockage de liquide de percolation.
